# EUROPEAN PATENT APPLICATION

(11) **EP 4 039 329 A1**
(43) Date of publication of application: **10.08.2022**
(21) Application number: 20871312.3
(22) Date of filing: 24.09.2020
(51) Int. Cl.: A61P 21/00, A61P 43/00, A23L 33/105, A61K 31/36

(54) **COMPOSITION FOR SUPPRESSION OF DECREASE IN MUSCLE MASS, PREVENTION OF DECREASE THEREIN, MAINTENANCE THEREOF, RECOVERY THEREOF OR INCREASE THEREIN**

(30) Priority: 04.10.2019 JP 2019183906
(71) Applicant: Suntory Holdings Limited, Osaka 530-8203 (JP)
(72) Inventor: YAGITA, Yuki, Soraku-gun, Kyoto 619-0284 (JP); ONO, Yoshiko, Soraku-gun, Kyoto 619-0284 (JP)
(74) Representative: Vossius & Partner Patentanwälte Rechtsanwälte mbB
(86) International application number: PCT/JP2020/035977
(87) International publication number: WO 2021/065661

(57) **Abstract**

The present invention aims to provide, for example, a composition for suppressing or preventing a decrease in muscle mass or maintaining, restoring, or increasing muscle mass, a composition for suppressing or preventing a decrease in blood albumin levels or maintaining or restoring blood albumin levels, a method of suppressing or preventing a decrease in muscle mass or maintaining, restoring, or increasing muscle mass, and a method of suppressing or preventing a decrease in blood albumin levels or maintaining or restoring blood albumin levels. The present invention relates to, for example, a composition for suppressing or preventing a decrease in muscle mass or maintaining, restoring, or increasing muscle mass, the composition containing at least one sesamin-class compound.

## Description

### TECHNICAL FIELD

The present invention relates to a composition for suppressing or preventing a decrease in muscle mass or maintaining, restoring, or increasing muscle mass. The present invention also relates to a composition for suppressing or preventing a decrease in blood albumin levels or maintaining or restoring blood albumin levels. The present invention also relates to, for example, a method of suppressing or preventing a decrease in muscle mass or maintaining, restoring, or increasing muscle mass; a method of suppressing or preventing a decrease in blood albumin levels or maintaining or restoring blood albumin levels; use of at least one sesamin-class compound for suppressing or preventing a decrease in muscle mass or maintaining, restoring, or increasing muscle mass; and use of at least one sesamin-class compound for suppressing or preventing a decrease in blood albumin levels or maintaining or restoring blood albumin levels.

### BACKGROUND ART

In a country like Japan where the ratio of elderly in the population is high, prolongation of health life expectancy has been a challenge. One of the factors that reduce the healthy life expectancy is aging-induced decrease in muscle mass. A decrease in muscle mass leads to a decrease in muscle strength, a decrease in walking speed, and the like, which may increase the risk of falling and cause injuries such as broken bones, and long-term bed rest, for example.

In addition, one of the causes that lead the elderly in the latter stage of life to be in need of nursing care is frailty from old age (Non-Patent Literature 1). Fried's definition is often used in diagnosis of frailty. According to the definition, a person is classified as frail if three or more of the following five symptoms are present, or as pre-frail if one or two of the following five symptoms are present: (1) weight loss, (2) malaise (fatigue), (3) decrease in activities, (4) decrease in muscle strength, and (5) decrease in walking speed (Non-Patent Literature 2). Sarcopenia is the state of decreased muscle mass, and is one of major risk factors of frailty.

While aging is the major cause of sarcopenia and frailty in the elderly, malnutrition is also one of risk factors. Patent Literature 1 discloses an agent for alleviating malnutrition, the agent containing a specific whey protein hydrolyzate as an active ingredient. According to the literature, the agent for alleviating malnutrition has an action to increase muscle mass and/or suppress a decrease in muscle mass, and an action to alleviate and/or prevent malnutrition that leads to low blood albumin or prealbumin levels. Albumin is a protein that binds to various molecules so as to transport these various molecules to various tissues. It has been reported that blood albumin levels serve as indices of progression of frailty and sarcopenia (Non-Patent Literatures 3 and 4).

### CITATION LIST

### - Patent Literature

Patent Literature 1: JP 2016-210725 A

### - Non-Patent Literature

Non-Patent Literature 1: Masafumi Kuzuya, Chokoreishakai ni okeru kyojaku no hyoka to kainyu no juyosei, Japan Medical Journal, 4599, 27-31, 2012
Non-Patent Literature 2: Fried LP et al., Frailty in older adults: evidence for a phenotype. J Gerontol A Biol Sci Med Sci 2001; 56: M146-156.
Non-Patent Literature 3: Chang CC et al., Association between low-grade albuminuria and frailty among community-dwelling middle-aged and older people: a cross-sectional analysis from I-Lan Longitudinal Aging Study. Sci Rep. 2016 Dec 21; 6: 39434.
Non-Patent Literature 4: Baumgartner RN et al., Serum albumin is associated with skeletal muscle in elderly men and women. Am J Clin Nutr. 1996; 64 (4) :552-558.

### SUMMARY OF INVENTION

### - Technical Problem

It is unknown that sesamin-class compounds such as sesamin have an action to suppress or prevent a decrease in muscle mass or maintain, restore, or increase muscle mass, and an action to suppress or prevent a decrease in blood albumin levels or maintain or restore blood albumin levels.

The present invention aims to provide, for example, a composition for suppressing or preventing a decrease in muscle mass or maintaining, restoring, or increasing muscle mass, and a composition for suppressing or preventing a decrease in blood albumin levels or maintaining or restoring blood albumin levels. The present invention also aims to provide, for example, a method of suppressing or preventing a decrease in muscle mass or maintaining, restoring, or increasing muscle mass, and a method of suppressing or preventing a decrease in blood albumin levels or maintaining or restoring blood albumin levels.

### - Solution to Problem

As a result of extensive studies to solve the above issue, the present inventors found that a sesamin-class compound has an action to suppress or prevent a decrease in muscle mass or maintain, restore, or increase muscle mass, and an action to suppress or prevent a decrease in blood albumin levels, or maintain or restore blood albumin levels.

The present invention relates to, for example, the following composition for suppressing or preventing a decrease in muscle mass or maintaining, restoring, or increasing muscle mass.
(1) A composition for suppressing or preventing a decrease in muscle mass or maintaining, restoring, or increasing muscle mass, the composition containing at least one sesamin-class compound.
(2) The composition according to (1) above, wherein the at least one sesamin-class compound includes at least one of sesamin or episesamin.
(3) The composition according to (1) or (2) above, wherein the at least one sesamin-class compound includes sesamin and episesamin, or episesamin.
(4) The composition according to any one of (1) to (3) above for use in preventing or reducing prefrailty or frailty.
(5) The composition according to any one of (1) to (4) above for use in preventing or reducing sarcopenia.
(6) A composition for suppressing or preventing a decrease in blood albumin levels or maintaining or restoring blood albumin levels, the composition containing at least one sesamin-class compound.
(7) The composition according to (6) above, wherein the at least one sesamin-class compound includes at least one of sesamin or episesamin.
(8) The composition according to (6) or (7) above, wherein the at least one sesamin-class compound includes sesamin and episesamin, or episesamin.
(9) The composition according to any one of (6) to (8) above for use in preventing or reducing prefrailty.
(10) The composition according to any one of (6) to (9) above for use in preventing or reducing sarcopenia.
(11) The composition according to any one of (1) to (10) above, wherein the composition is an oral composition.
(12) The composition according to any one of (1) to (11) above, wherein the composition is in the form of a food or beverage.
(13) A method of suppressing or preventing a decrease in muscle mass or maintaining, restoring, or increasing muscle mass, the method including administering at least one sesamin-class compound.
(14) A method of suppressing or preventing a decrease in blood albumin levels or maintaining or restoring blood albumin levels, the method including administering at least one sesamin-class compound.
(15) Use of at least one sesamin-class compound for suppressing or preventing a decrease in muscle mass or maintaining, restoring, or increasing muscle mass.
(16) Use of at least one sesamin-class compound for suppressing or preventing a decrease in blood albumin levels or maintaining or restoring blood albumin levels.

### - Advantageous Effects of Invention

The present invention can provide, for example, a composition for suppressing or preventing a decrease in muscle mass or maintaining, restoring, or increasing muscle mass, and a composition for suppressing or preventing a decrease in blood albumin levels or maintaining or restoring blood albumin levels. The present invention can also provide a method of suppressing or preventing a decrease in muscle mass or maintaining, restoring, or increasing muscle mass. The present invention can provide a method of suppressing or preventing a decrease in blood albumin levels or maintaining or restoring blood albumin levels.

Ingestion of at least one sesamin-class compound provides an effect of suppressing or preventing a decrease in muscle mass or maintaining, restoring, or increasing muscle mass. Ingestion of at least one sesamin-class compound provides an effect of suppressing or preventing a decrease in blood albumin levels or maintaining or restoring blood albumin levels. For example, prefrailty, frailty, sarcopenia, or the like can be prevented or reduced by suppressing or preventing a decrease in muscle mass, maintaining muscle mass, restoring decreased muscle mass, or increasing muscle mass. Prefrailty, frailty, sarcopenia, or the like can also be prevented or reduced by suppressing or preventing a decrease in blood albumin levels, maintaining blood albumin levels, or restoring decreased blood albumin levels. The sesamin-class compound for use in the present invention is a component that has a long history of consumption as food, and is advantageous in that it is highly safe and can be ingested continuously.

### BRIEF DESCRIPTION OF DRAWINGS

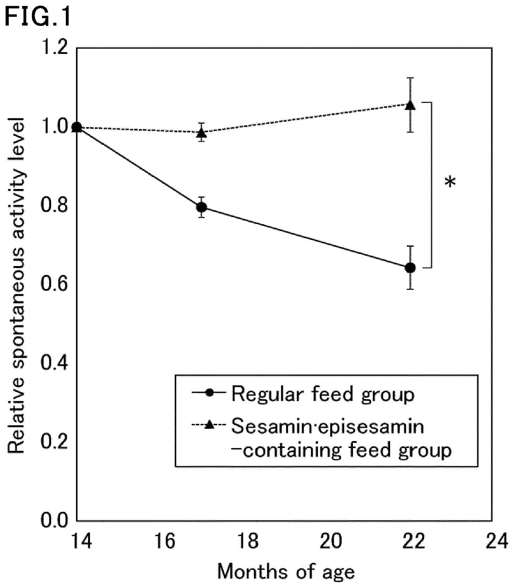

Fig. 1 is a graph showing an effect of a mixture of sesamin and episesamin (sesamin:episesamin = 1:1 by weight) on spontaneous activity level.

### DESCRIPTION OF EMBODIMENTS

The composition for suppressing or preventing a decrease in muscle mass or maintaining, restoring, or increasing muscle mass of the present invention contains at least one sesamin-class compound.

The composition for suppressing or preventing a decrease in blood albumin levels or maintaining or restoring blood albumin levels of the present invention contains at least one sesamin-class compound.

The composition for suppressing or preventing a decrease in muscle mass or maintaining, restoring, or increasing muscle mass and the composition for suppressing or preventing a decrease in blood albumin levels or maintaining or restoring blood albumin levels each contain at least one sesamin-class compound as an active ingredient. The term "muscle mass" usually refers to skeletal muscle mass. The term "muscle mass" may refer to muscle weight.

The primary cause of frailty and sarcopenia is aging. Changes with aging are caused by complex influence of various factors. Models reproduced by genetic engineering and/or drug administration merely focus on limited factors of frailty and/or sarcopenia.

As described in Examples (described later), when aged animals were continuously fed with sesamin and episesamin, these aged animals had more muscle mass than animals not fed with sesamin or episesamin. In addition, aging-induced decrease in blood albumin levels was suppressed because of ingestion of sesamin and episesamin. This suggested that ingestion of a sesamin-class compound can suppress aging-induced decrease in blood albumin levels or maintain muscle mass, for example.

The sesamin-class compound has an action to suppress or prevent a decrease in muscle mass or maintain, restore, or increase muscle mass, and can be used to suppress a decrease in muscle mass, prevent a decrease in muscle mass, maintain muscle mass, restore muscle mass, or increase muscle mass.

The sesamin-class compound also has an action to suppress or prevent a decrease in blood albumin levels or maintain or restore blood albumin levels, and can be used to suppress a decrease in blood albumin levels, prevent a decrease in blood albumin levels, maintain blood albumin levels, or restore blood albumin levels. The terms "restore" and "restoring" encompass at least partial restoration.

The sesamin-class compound is likely to contribute to preventing or reducing prefrailty, frailty, sarcopenia, or the like by its action to suppress or prevent a decrease in muscle mass or maintain, restore, or increase muscle mass and/or its action to suppress or prevent a decrease in blood albumin levels or maintain or restore blood albumin levels. The term "frailty" refers to physical frailty. Physical frailty may include a decrease in muscle mass and/or muscle strength. Prefrailty may include a decrease in muscle mass and/or muscle strength. Prevention or reduction of sarcopenia is effective in preventing or reducing frailty, prefrailty, or the like that includes a decrease in muscle mass and/or muscle strength, for example.

The composition for suppressing or preventing a decrease in muscle mass or maintaining, restoring, or increasing muscle mass of the present invention contains at least one sesamin-class compound as an active ingredient and thereby achieves an excellent effect of suppressing or preventing a decrease in muscle mass or maintaining, restoring, or increasing muscle mass. Such an effect makes is possible, for example, to suppress or prevent a decrease in muscle strength, maintain muscle strength, restore decreased muscle strength, and alleviate a condition in which muscle mass or muscle strength has decreased. In one embodiment, the composition for suppressing or preventing a decrease in muscle mass or maintaining, restoring, or increasing muscle mass of the present invention is suitably used for suppressing or preventing a decrease in muscle mass or maintaining or restoring muscle mass. In one embodiment, the composition for suppressing or preventing a decrease in muscle mass or maintaining, restoring, or increasing muscle mass of the present invention is suitably used for suppressing or preventing aging-induced decrease in muscle mass or maintaining muscle mass, or restoring or increasing muscle mass that has decreased due to aging. The muscle mass may be leg muscle mass, for example. In one embodiment, the composition is suitably used by middle-aged and older people for suppressing or preventing a decrease in aging-induced decrease in muscle mass or maintaining muscle mass, or restoring or increasing muscle mass that has decreased due to aging. The term "middle-aged and older people" is a concept that includes the elderly.

The muscle mass of animals including humans can be measured by, for example, microPET/CT (positron emission tomography/computed tomography, INVEON, Siemens, USA).

The composition for suppressing or preventing a decrease in muscle mass or maintaining, restoring, or increasing muscle mass of the present invention can be used for treating conditions or diseases that are likely to be prevented or alleviated by suppressing or preventing a decrease in muscle mass or maintaining, restoring, or increasing muscle mass. Examples of such conditions or diseases include conditions or diseases with decreased muscle mass or decreased muscle strength, such as prefrailty, frailty, or sarcopenia. In one embodiment, the composition for suppressing or preventing a decrease in muscle mass or maintaining, restoring, or increasing muscle mass may be used for preventing or reducing prefrailty, frailty, or sarcopenia.

The terms "prevention" and "preventing" as used herein encompass preventing disease or symptom onset, delaying disease or symptom onset, decreasing disease or symptom incidence, decreasing risk of disease or symptom onset, and the like. The phrase "alleviating conditions or diseases" encompasses helping the subject recover from conditions or diseases, alleviating conditions or disease symptoms, making positive changes in conditions or disease symptoms, delaying or preventing progression of conditions or diseases, and the like.

The composition for suppressing or preventing a decrease in blood albumin levels or maintaining or restoring blood albumin levels of the present invention contains at least one sesamin-class compound as an active ingredient and thereby achieves an excellent effect of suppressing or preventing a decrease in blood albumin levels or maintaining or restoring blood albumin levels. The composition for suppressing or preventing a decrease in blood albumin levels or maintaining or restoring blood albumin levels of the present invention can be used for treating conditions or diseases that are likely to be prevented or alleviate by suppressing or preventing a decrease in blood albumin levels or maintaining or restoring blood albumin levels. Examples of such conditions or diseases include malnutrition, preferably malnutrition that leads to low blood albumin levels. The composition for suppressing or preventing a decrease in blood albumin levels or maintaining or restoring blood albumin levels of the present invention can be used to prevent or alleviate malnutrition that leads to low blood albumin levels. Examples of malnutrition that leads to low blood albumin levels include prefrailty, frailty, and sarcopenia. In one embodiment, the composition for suppressing or preventing a decrease in blood albumin levels or maintaining or restoring blood albumin levels of the present invention can be used for suppressing or preventing aging-induced decrease in blood albumin levels, maintaining blood albumin levels, or restoring blood albumin levels that have decreased due to aging. In one embodiment, the composition is suitably used by middle-aged and older people for suppressing or preventing aging-induced decrease in blood albumin levels, maintaining blood albumin levels, or restoring blood albumin levels that have decreased due to aging.

The composition for suppressing or preventing a decrease in muscle mass or maintaining, restoring, or increasing muscle mass of the present invention, and the composition for suppressing or preventing a decrease in blood albumin levels or maintaining or restoring blood albumin levels are sometimes also collectively referred to as "the composition of the present invention".

In the present invention, the term "sesamin-class compound" is the collective term for compounds including sesamin and its analogs. Sesamin is one of main lignan compounds found in sesame. Examples of the sesamin analogs include episesamin and dioxabicyclo[3.3.0]octane derivatives described in JP H04-9331 A. The at least one sesamin-class compound may include a single compound alone or two or more compounds. Specific examples of the sesamin-class compound include sesamin, episesamin, sesaminol, episesaminol, sesamol, and sesamolin. Stereoisomers or racemates of these compounds may be used alone or in mixture. In addition, metabolites of the sesamin-class compound (e.g., those described in JP 2001-139579 A) are also sesamin analogs included in the sesamin-class compound of the present invention, and can also be used in the present invention, as long as they exhibit the effect of the present invention. In the present invention, sesamin and/or episesamin can be suitably used as the at least one sesamin-class compound. Sesamin and episesamin, or episesamin can be more suitably used, and sesamin and episesamin can be even more suitably used. When sesamin and episesamin are used, the ratio of these components is not limited. For example, the ratio of sesamin to episesamin by weight is preferably 1:0.1 to 1:9, more preferably 1:0.3 to 1:3, still more preferably 1:0.5 to 1:2. In one embodiment, use of episesamin as the sesamin-class compound is more preferred.

The sesamin-class compound for use in the present invention is not limited in any way by its form, production method, or the like. For example, when the sesamin-class compound is sesamin, sesamin extracted from sesame oil by a known method (e.g., the method described in JP H04-9331 A) can be used (hereinafter, such sesamin is called a sesamin extract or purified sesamin). Commercially available sesame oil (in liquid form) can also be used as is. However, when sesame oil is used, the characteristic flavor of sesame oil is sometimes evaluated as being organoleptically undesirable. Thus, a tasteless and odorless sesamin extract (or purified sesamin) from sesame oil is preferably used. In addition, since sesame oil has a low sesamin content, use of sesame oil to incorporate a desired amount of sesamin results in excess volume per unit dosage of a composition to be prescribed. This sometimes causes inconvenience in ingestion. In particular, in the case where the composition is formulated for oral administration, the preparation (e.g., tablet or capsule) becomes so bulky that it causes trouble in ingestion. Thus, use of sesamin extract (or purified sesamin) from sesame oil is preferred because it does not require a large amount of ingestion. In the present invention, at least one purified or isolated sesamin-class compound may be used. The sesamin-class compound can also be obtained by synthesis. For example, sesamin and episesamin can be synthesized by the method of Beroza et al. (J. Am. Chem. Soc., 78, 1242 (1956)). Metabolites of sesamin and episesamin can be synthesized by the method of Urata et al. (Chem. Pharm. Bull. (Tokyo), 56(11), 1611-2 (2008)).

The sesamin-class compound is a compound that is found in natural products and food and beverages and that has a history of consumption as food. Thus, the sesamin-class compound is less likely to cause problems in terms of safety, for example, even if ingested daily. The present invention can provide a composition for suppressing or preventing a decrease in muscle mass or maintaining, restoring, or increasing muscle mass, and a composition for suppressing or preventing a decrease in blood albumin levels or maintaining or restoring blood albumin levels, each composition containing a highly safe material as an active ingredient.

The composition of the present invention is applicable for either therapeutic use (medical use) or non-therapeutic use (non-medical use). The "non-therapeutic"
is a concept that does not include medical activities, i.e., a concept that does not include methods of surgery, therapy, or diagnosis of humans.

The composition of the present invention can be provided in the form of a food or beverage, a pharmaceutical product, a quasi-pharmaceutical product, feed, or the like. The composition of the present invention may be a food or beverage, a pharmaceutical product, a quasi-pharmaceutical product, feed, or the like to be used for suppressing or preventing a decrease in muscle mass or maintaining, restoring, or increasing muscle mass or for suppressing or preventing a decrease in blood albumin levels or maintaining or restoring blood albumin levels by itself; or the composition may be a formulation, material, or the like to be added to these products.

The composition of the present invention can be provided in the form of an agent for example, but it is not limited thereto. Such an agent may be provided as a composition as is, or as a composition containing the agent.

The composition of the present invention may be either an oral composition or a parenteral composition, but it is preferably an oral composition. The oral composition may be a food or beverage, an oral pharmaceutical product, an oral quasi-pharmaceutical product, or feed, preferably a food or beverage or an oral pharmaceutical product, more preferably a food or beverage.

The composition of the present invention can contain optional additives and optional ingredients in addition to the at least one sesamin-class compound, as long as the effect of the present invention is not impaired. Such additives and ingredients may be selected depending on the form of the composition, for example, and those generally usable in food and beverages, pharmaceutical products, quasi-pharmaceutical products, feed, and the like can be used. When the composition of the present invention is provided as a food or beverage, a pharmaceutical product, a quasi-pharmaceutical product, feed, or the like, any common method can be used for the production.

For example, when the composition of the present invention is provided as a food or beverage, ingredients usable in food and beverages (e.g., food materials and optional food additives) can be added to the at least one sesamin-class compound to provide various food and beverages. The food or beverage is not limited. Examples thereof include general food and beverages, health foods, dietary supplements, health drinks, foods with function claims, foods for specified health uses, and foods for the sick. The health foods, dietary supplements, foods with function claims, foods for specified health uses, and the like can be used in various forms of formulations such as fine granules, tablets, granules, powders, capsules, chewable tablets, dry syrups, syrups, liquid agents, beverages, and liquid foods.

When the composition of the present invention is provided as a pharmaceutical product or a quasi-pharmaceutical product, for example, a pharmacologically acceptable carrier, an optional additive, or the like can be added to the at least one sesamin-class compound to provide various dosage forms of pharmaceutical products or quasi-pharmaceutical products. Such a carrier, an additive, or the like may be any pharmacologically acceptable one that can be used in pharmaceutical products or quasi-pharmaceutical products. Examples thereof include excipients, binders, disintegrants, lubricants, antioxidants, and colorants. One or more of these can be used. The form of administration (ingestion) of the pharmaceutical product or the quasi-pharmaceutical product may be oral or parenteral (e.g., transdermal, transmucosal, enteral, or injection) administration. When the composition of the present invention is provided as a pharmaceutical product or a quasi-pharmaceutical product, it is preferably an oral pharmaceutical product or a quasi-pharmaceutical product. Examples of the dosage form for oral administration include liquids, tablets, powders, fine granules, granules, sugar-coated tablets, capsules, suspensions, emulsions, and chewable tablets. The pharmaceutical product may be for non-human animals.

When the composition of the present invention is provided as feed, at least one sesamin-class compound is simply added to feed. The feed includes feed additives. Examples of the feed include feed for farm animals such as cows, pigs, chickens, sheep, and horses; feed for small animals such as rabbits, rats, and mice; and pet food for animals such as dogs, cats, and birds.

The amount of the sesamin-class compound in the composition of the present invention is not limited, and can be set according to the form of the composition or the like.

The total amount of the sesamin-class compound in the composition of the present invention is, for example, preferably 0.001 wt% or more, more preferably 0.01 wt% or more, still more preferably 0.05 wt% or more and is preferably 10 wt% or less, more preferably 5 wt% or less. In one embodiment, the total amount of the sesamin-class compound in the composition is preferably 0.001 to 10 wt%, more preferably 0.01 to 5 wt%, still more preferably 0.05 to 5 wt%. When the composition contains two or more sesamin-class compounds, the total amount is the sum of these compounds.

Preferably, the composition of the present invention is orally ingested or orally administered. The intake (which can also be referred to as "dosage") of the composition of the present invention is not limited. The intake of the composition of the present invention may be any amount that achieves an effect of suppressing or preventing a decrease in muscle mass or maintaining, restoring, or increasing muscle mass, or an effect of suppressing or preventing a decrease in blood albumin levels or maintaining or restoring blood albumin levels. The intake may be appropriately set according to the administration form, administration method, body weight of a subject, and the like.

In one embodiment, when the composition of the present invention is orally fed or administered to a human (adult), the total intake of the sesamin-class compound is preferably 0.5 mg or more, more preferably 1 mg or more, still more preferably 3 mg or more, and is preferably 200 mg or less, more preferably 100 mg or less, still more preferably 80 mg or less per 60 kg body weight per day. In one embodiment, the total intake of the sesamin-class compound is preferably 0.5 to 200 mg, more preferably 1 to 100 mg, still more preferably 3 to 80 mg per 60 kg body weight per day in the case of a human (adult). Preferably, the above amount of the composition is fed or administered in at least one portion per day, for example, in one to several portions (e.g., two or three portions) per day. In one embodiment, preferably, the above amount of the sesamin-class compound is orally fed or administered to a human. In one embodiment, the composition of the present invention can be used to feed or administer the above amount of the sesamin-class compound to a human per 60 kg body weight per day. When the composition contains two or more sesamin-class compounds, the total intake is the sum of these compounds. In one embodiment, preferably, sesamin and/or episesamin is orally fed or administered to a human (adult) in an amount of preferably 0.5 to 200 mg, more preferably 1 to 100 mg, still more preferably 3 to 80 mg per 60 kg body weight per day as the total intake of sesamin and episesamin.

Preferably, the composition of the present invention is continuously fed or administered. The above effects are likely to be enhanced when the sesamin-class compound is continuously fed or administered. In one embodiment, the composition of the present invention is continuously fed or administered for preferably one week or more, more preferably four weeks or more, still preferably eight weeks or more, particularly preferably 12 weeks or more.

The subject to which the composition of the present invention is fed or administered (which can also be referred to as an "administration subject") is not limited. The administration subject is preferably a human or non-human mammal, more preferably a human.

In one embodiment, the administration subject may be one who needs or wants to suppress or prevent a decrease in muscle mass or maintain, restore, or increase muscle mass; or one who needs or wants to suppress or prevent a decrease in blood albumin levels or maintain or restore blood albumin levels. Examples of such a subject include middle-aged and older people, a subject who needs or wants to prevent or reduce frailty or sarcopenia, a subject who needs or wants to prevent or alleviate malnutrition, and a subject who needs or wants to prevent or alleviate physical weakness. Examples of the administration subject also include a patient with frailty, a patient with prefrailty, a patient with sarcopenia, and a patient with malnutrition. The middle-aged and older people may be those who are 40 years old or older, for example. In one embodiment, the elderly are preferred subjects among the middle-aged and older people. The elderly may be people who are 60 years old or older or 65 years old or older, for example. For example, the composition of the present invention can be used by healthy people for purposes such as preventing symptoms or diseases that are likely to be prevented or alleviated by suppressing or preventing a decrease in muscle mass, or maintaining, restoring, or increasing muscle mass, suppressing or preventing a decrease in blood albumin levels, or maintaining or restoring blood albumin levels.

The composition for suppressing or preventing a decrease in muscle mass or maintaining or increasing muscle mass of the present invention may be labeled with a function claim for an effect that may be exerted by suppressing or preventing a decrease in muscle mass or maintaining, restoring, or increasing muscle mass. For example, the composition may be labeled with one or more function claims selected from the group consisting of "suppressing aging-induced decrease in muscle mass", "maintaining muscle mass needed to live an independent daily life", "suppressing a decrease in muscle mass and muscle strength needed to live a daily life", "maintaining strength to support the body", "maintaining muscle that gets weak due to aging", "maintaining walking ability or walking function", and "suppressing a decrease in walking ability or walking function".

The composition for suppressing or preventing a decrease in blood albumin levels or maintaining or restoring blood albumin levels of the present invention may be labeled with a function claim for an effect that may be achieved by suppressing or preventing a decrease in blood albumin levels or maintaining or restoring blood albumin levels. For example, the composition may be labeled with one or more function claims selected from the group consisting of "alleviating physical weakness" and "alleviating malnutrition".

In one embodiment of the present invention, preferably, the composition of the present invention is a food or beverage labeled with one or more function claims described above. These labels may be labels indicating use for obtaining these functions.

The present invention also encompasses the following methods:
a method of suppressing or preventing a decrease in muscle mass or maintaining, restoring, or increasing muscle mass, the method including administering at least one sesamin-class compound; and
a method of suppressing or preventing a decrease in blood albumin levels or maintaining or restoring blood albumin levels, the method including administering at least one sesamin-class compound.

The present invention also encompasses the following uses:
use of at least one sesamin-class compound for suppressing or preventing a decrease in muscle mass or maintaining, restoring, or increasing muscle mass; and
use of at least one sesamin-class compound for suppressing or preventing a decrease in blood albumin levels or maintaining or restoring blood albumin levels.

The above uses and the methods may be therapeutic or non-therapeutic.

Administration of at least one sesamin-class compound to a subject can suppress or prevent a decrease in muscle mass or maintain, restore, or increase muscle mass. This makes it possible, for example, to prevent or reduce sarcopenia, prefrailty, frailty, or the like.

Administration of at least one sesamin-class compound to a subject can suppress or prevent a decrease in blood albumin levels or maintain or restore blood albumin levels. This makes it possible, for example, to prevent or alleviate malnutrition (preferably, malnutrition that leads to low blood albumin levels), sarcopenia, prefrailty, frailty, or the like.

The present invention also encompasses a method of preventing or reducing sarcopenia, prefrailty, or frailty, the method including administering at least one sesamin-class compound. The present invention also encompasses a method of preventing or alleviating malnutrition, the method including administering at least one sesamin-class compound.

In the above methods and uses, the sesamin-class compound and its preferred embodiments are as described above for the composition of the present invention. The at least one sesamin-class compound may include one sesamin-class compound or two or more sesamin-class compounds. In the above methods and uses, preferably, the at least one sesamin-class compound is administered (fed) to a subject at least once a day, for example, one to several times (e.g., two to three times) a day. In the above methods and uses, preferably, the sesamin-class compound is orally administered (fed). The above uses are preferably for humans or non-human mammals, more preferably for humans.

The above methods and uses only require use of at least one sesamin-class compound in an amount (effective amount) that achieves a desired effect(s). Preferred intake, preferred administration subjects, and the like of the sesamin-class compound are as described above for the composition of the present invention. The sesamin-class compound may be administered as is, or may be administered in the form of a composition containing the sesamin-class compound. For example, the above-described composition of the present invention may be used.

The present invention also encompasses use of at least one sesamin-class compound for producing the composition of the present invention.

All academic literature and patent literature cited herein are incorporated herein by reference.

### EXAMPLES

The present invention is described in further detail below with reference to examples. A series of animal experiments was performed based on a plan approved by the relevant chief through evaluation of the in-house animal experiment committee, in compliance with the animal welfare management laws and other related laws and regulations. The present invention is not limited to these examples.

### <Example 1: Effects of 1:1 mixture of sesamin and episesamin on muscle mass and on suppression of decrease in albumin levels (in vivo test)>

Old rats (14 months of age) were used to examine an effect of a mixture of sesamin and episesamin on muscle mass and an effect of the mixture to suppress a decrease in albumin levels. The rats were divided into a group to be fed with regular feed and a group to be fed with a mixture of sesamin and episesamin in such a manner that each group would have the same spontaneous activity level. The spontaneous activity level was measured using an accelerometer nanotag (available from Kissei Comtec Co., Ltd.). An accelerometer was embedded in the skin of the neck region of each male Slc:SD rat (14 months of age) under isoflurane anesthesia. After recovery was confirmed, the spontaneous activity level in the active period (dark period) was measured for three days. To measure the spontaneous activity level, the number of vibrations of a certain intensity or higher was continuously counted, and the counts were summed to obtain the spontaneous activity level. The rats were divided into a group to be fed with regular feed (AIN-93M available from CLEA Japan, Inc.) and a group to be fed with feed prepared by mixing the regular feed with 0.1 wt% sesamin and episesamin (sesamin:episesamin = 1:1 by weight) (8 rats per group). Each group was fed with its corresponding feed (test diet) for eight months. The feed containing sesamin and episesamin were prepared by mixing the regular feed with a mixture of sesamin and episesamin (sesamin:episesamin = 1:1 by weight). The group fed with the regular feed is referred to as the old regular feed group (regular feed group); the group fed with the feed containing 0.1 wt% sesamin and episesamin (sesamin·episesamin mixture group) is referred to as the old sesamin·episesamin-containing feed group (sesamin·episesamin-containing feed group). The feed containing sesamin and episesamin had a total amount of sesamin and episesamin of 0.1 wt%. The spontaneous activity level was measured for one week at 17 months of age and 22 months of age. Blood was collected under isoflurane anesthesia from 22-month-old animals eight months after starting ingestion of the test diet. After the animals were euthanized by phlebotomy, the gastrocnemius muscle, tibialis anterior muscle, and soleus muscle were collected. Blood albumin levels were measured using the collected blood. In order to understand the health conditions of the animals, the amount of feed and body weight of each animal were measured regularly. A rat determined as inappropriate for further testing was euthanized in an appropriate manner and excluded from analysis. The subjects of analysis were ultimately four rats in the old regular feed group and six rats in the old sesamin·episesamin-containing feed group.

As a young regular feed group, male Slc:SD rats (4 months of age) were fed with regular feed (AIN-93M available from CLEA Japan, Inc.) for four weeks (6 rats/group). Blood was collected under isoflurane anesthesia. Blood albumin levels were measured using the collected blood.

Table 1 shows the weight of each of gastrocnemius muscle, tibialis anterior muscle, and soleus muscle (average ± standard error). The old sesamin·episesamin-containing feed group showed a higher muscle weight than the old regular feed group.

**[Table 1]**

| Group name | Gastrocnemius muscle (g) | Tibialis anterior muscle (g) | Soleus muscle (g) |
|---|---|---|---|
| Old regular feed group | 1.987 ± 0.131 | 0.817 ± 0.055 | 0.184 ± 0.008 |
| Old sesamin episesamin-containing feed group | 2.274 ± 0.032 | 0.898 ± 0.020 | 0.211 ± 0.011 |

Table 2 shows blood albumin levels (average ± standard error). As shown in Table 2, the old regular feed group showed lower blood albumin levels than the young regular feed group, and the old sesamin·episesamin-containing feed group showed higher blood albumin levels than the old regular feed group.

**[Table 2]**

| Group name | Blood albumin levels (g/dL) |
|---|---|
| Young regular feed group | 3.99 ± 0.19 |
| Old regular feed group | 2.81 ± 0.22 |
| Old sesamin·episesamin-containing feed group | 3.40 ± 0.12 |

Fig. 1 shows changes in the spontaneous activity level (relative spontaneous activity level), taking the spontaneous activity level before starting test diet as 1. In the graph in Fig. 1, the circles (●) indicate the old regular feed group, and the triangles (▲) indicate the old sesamin·episesamin-containing feed group (*: p < 0.05 vs. old regular feed group, repeated measures two-way ANOVA). A lower value indicates a lower activity level. As shown in Fig. 1, the regular feed group showed a decrease in spontaneous activity level with aging. In contrast, the sesamin·episesamin-containing feed group showed suppression of aging-induced decrease in spontaneous activity level. Repeated measurement analysis showed an interaction between test diet and time (p = 0.014) in the sesamin·episesamin-containing feed group. At 22 months of age, the sesamin·episesamin-containing feed group showed a significantly higher spontaneous activity level than the regular feed group (Bonferroni test, p = 0.023).

### <Example 2: Effect of administration of sesamin or episesamin alone on suppression of decrease in spontaneous activity level (in vivo test)>

Example 2 is a test performed to examine which one of sesamin or episesamin has a greater contribution to suppression of a decrease in activity level. The basic operation was the same as that in Example 1.

Male Slc:SD rats (14 months of age) were divided into a group to be fed with regular feed (CE-2 available from CLEA Japan, Inc.), a group to be fed with feed containing 0.1% sesamin, and a group to be fed with 0.1% episesamin (3 rats/group) in such a manner each group would have the same activity level. Each group was fed with its corresponding feed (test diet) for one month. The rats were subjected to measurement of spontaneous activity level in the active period at 16 months of age one month after ingestion of the test diet for one month. The subjects of analysis were ultimately two rats in the regular feed group, three rats in the sesamin-containing feed group, and three rats in the episesamin-containing feed group.

Table 3 shows the spontaneous activity level of each group after fed with the test diet for one month, taking the spontaneous activity level before starting the test diet as 1. A lower value indicates a lower activity level. As shown in Table 3, the regular feed group showed a decrease in spontaneous activity level. In contrast, the sesamin-containing feed group and the episesamin-containing feed group showed suppression of aging-induced decrease in spontaneous activity level, compared to the regular feed group.

**[Table 3]**

| Group name | Activity level, taking activity level before starting test diet as 1 |
|---|---|
| Regular feed group | 0.89 |
| Sesamin group | 0.91 |
| Episesamin group | 1.07 |

The above suggests that sesamin and episesamin suppressed aging-induced decrease in muscle mass and albumin levels and thereby suppressed or reduced prefrailty with a decreased activity level.

## Claims

1. A composition for suppressing or preventing a decrease in muscle mass or maintaining, restoring, or increasing muscle mass, the composition comprising:
at least one sesamin-class compound.

2. The composition according to claim 1, wherein the at least one sesamin-class compound includes at least one of sesamin or episesamin.

3. The composition according to claim 1 or 2, wherein the at least one sesamin-class compound includes sesamin and episesamin, or episesamin.

4. The composition according to any one of claims 1 to 3 for use in preventing or reducing prefrailty or frailty.

5. The composition according to any one of claims 1 to 4 for use in preventing or reducing sarcopenia.

6. A composition for suppressing or preventing a decrease in blood albumin levels or maintaining or restoring blood albumin levels, the composition comprising:
at least one sesamin-class compound.

7. The composition according to claim 6, wherein the at least one sesamin-class compound includes at least one of sesamin or episesamin.

8. The composition according to claim 6 or 7, wherein the at least one sesamin-class compound includes sesamin and episesamin, or episesamin.

9. The composition according to any one of claims 6 to 8 for use in preventing or reducing prefrailty.

10. The composition according to any one of claims 6 to 9 for use in preventing or reducing sarcopenia.

11. The composition according to any one of claims 1 to 10,
wherein the composition is an oral composition.

12. The composition according to any one of claims 1 to 11,
wherein the composition is in the form of a food or beverage.

13. A method of suppressing or preventing a decrease in muscle mass or maintaining, restoring, or increasing muscle mass, the method comprising:
administering at least one sesamin-class compound.

14. A method of suppressing or preventing a decrease in blood albumin levels or maintaining or restoring blood albumin levels, the method comprising:
administering at least one sesamin-class compound.

15. Use of at least one sesamin-class compound for suppressing or preventing a decrease in muscle mass or maintaining, restoring, or increasing muscle mass.

16. Use of at least one sesamin-class compound for suppressing or preventing a decrease in blood albumin levels or maintaining or restoring blood albumin levels.
